# EUROPEAN PATENT APPLICATION

(11) **EP 4 813 521 A2**
(43) Date of publication of application: **30.09.2026**
(21) Application number: 26166832.1
(22) Date of filing: 23.03.2026
(51) Int. Cl.: B05B 1/18

(54) **SHOWERHEAD INTEGRATED WITH A CENTRAL PHOTOTHERAPY UNIT**

(30) Priority: 25.03.2025 CN 202520531186 U; 22.12.2025 CN 202522719493 U
(71) Applicant: Shenzhen Rainbow Light Medical Equipment Co., Ltd., Shenzhen, Guangdong 518103 (CN); Higherdose LLC, New York, NY 10004 (US)
(72) Inventor: BERLINGERI, Lauren, New York, NY 10004 (US); DIJKSTRA, Alain, 1186 GK Amstelveen (NL); JIAMING, Cao, Shenzhen, 518103 (CN); XIANG, Huang, Shenzhen, 518103 (CN); ZUN, Qui, Shenzhen, 518103 (CN); GUANG, Tian, Shenzhen, 518103 (CN)
(74) Representative: Valet Patent Services Limited

(57) **Abstract**

Embodiments of the present invention relate to a multifunctional showerhead incorporating a phototherapy unit. The showerhead comprises a showerhead body including a housing defining a water cavity, a water outlet side, and a mounting hole, and a phototherapy unit detachably disposed within the mounting hole. The phototherapy unit is configured to emit therapeutic light toward a user during shower operation, enabling simultaneous showering and phototherapy. The phototherapy unit may emit light of one or more wavelengths and may include optical structures for improving light distribution uniformity. Various embodiments provide detachable locking structures, multiple power supply modes, control and switching mechanisms, and waterproof and electrical safety features. The showerhead may further include smart control functions, user interface indicators, and hygiene and maintenance enhancements.

## Description

### TECHNICAL FIELD

The present invention relates to the field of bathing and personal care devices, and more particularly to a showerhead incorporating a phototherapy unit. The invention specifically concerns a showerhead structure configured to accommodate a detachable or mountable phototherapy unit. The phototherapy unit is capable of emitting therapeutic light toward a user during showering, thereby combining conventional water delivery functionality with light-based therapeutic treatment.

### BACKGROUND ART

Conventional showerheads available in the market are primarily designed to perform a single function, namely, delivering water for bathing purposes. Such traditional showerheads typically include a housing, a water inlet, and a plurality of water outlet holes for spraying water onto a user. While variations exist in spray patterns, water pressure regulation, or filtration features, the core functionality of these devices remains limited to basic cleansing. As a result, traditional showerheads do not provide any additional health, wellness, or therapeutic benefits beyond routine bathing.

In recent years, phototherapy technologies such as red light, blue light, or other wavelength-based light therapies have gained attention for their potential benefits in skin care, muscle relaxation, circulation improvement, and overall wellness. The benefit of taking phototherapy while bathing is the localized, enhanced delivery of the photosensitizing agent to the skin, which increase the treatment effectiveness for skin conditions, while potentially reducing systemic side effects associated with oral medicine. However, existing phototherapy devices are generally implemented as standalone units, handheld devices, or fixed installations separate from bathing appliances. These conventional phototherapy solutions often require additional installation space, separate power arrangements, and independent operation, which can be inconvenient for users and limit widespread adoption in daily routines.

Accordingly, there exists a gap in the market for a bathing device that seamlessly integrates phototherapy functionality with a showerhead structure, without significantly increasing installation complexity or altering established user habits. In particular, there is a need for a solution that conveniently delivers phototherapy during normal showering while maintaining structural compactness, safety, and ease of maintenance. Furthermore, existing solutions lack modularity, making replacement, repair, or customization of phototherapy units difficult or impractical.

The present invention addresses the above-mentioned shortcomings by providing a showerhead incorporating a phototherapy unit that is detachably or removably disposed within a mounting portion of the showerhead body. The phototherapy unit is configured to emit therapeutic light outwardly toward the user during shower operation, thereby enabling simultaneous showering and phototherapy. By integrating the phototherapy unit directly into the showerhead structure, the invention eliminates the need for separate phototherapy devices and allows users to receive therapeutic benefits as part of their regular bathing routine.

Moreover, the detachable configuration of the phototherapy unit offers significant advantages, including ease of installation, maintenance, and replacement. Users may conveniently remove or replace the phototherapy unit in the event of malfunction, when upgrading, or when selecting different light therapy modes, without replacing the entire showerhead. The invention thus enhances functionality while preserving structural simplicity and user convenience.

Overall, the disclosed showerhead provides a practical, space-efficient, and multifunctional solution that bridges the gap between conventional bathing appliances and phototherapy technology, offering improved user experience, enhanced wellness benefits, and greater versatility compared to traditional showerheads and existing phototherapy devices.

### OBJECTS OF THE INVENTION

Some of the objects of the invention are as follows:
An object of the present invention is to provide a showerhead integrating a phototherapy unit that enables simultaneous showering and delivery of therapeutic light to a user during bathing.

Another object of the present invention is to provide a phototherapy-integrated showerhead having a detachable phototherapy unit, thereby facilitating easy installation, removal, replacement, and maintenance of the phototherapy unit.

A further object of the present invention is to provide a showerhead in which the phototherapy unit is securely mounted within a mounting hole using reliable locking structures, such as snap-fit, locking groove, or magnetic connection mechanisms, to ensure stability during use.

Another object of the present invention is to provide a showerhead capable of emitting therapeutic light of one or more wavelengths, such as red light, blue light, or near-infrared light, to achieve different phototherapy effects for skin care, relaxation, or wellness applications.

Another object of the present invention is to provide a compact and integrated showerhead structure in which water outlet holes are arranged around the phototherapy unit, allowing water flow and therapeutic light to act simultaneously and uniformly on the user's body.

A still further object of the present invention is to provide a showerhead with adjustable orientation through a rotatable ball joint, enabling users to conveniently adjust both water spray direction and light emission direction.

Yet another object of the present invention is to provide a safe, reliable, and industrially applicable phototherapy-integrated showerhead that enhances user experience, improves therapeutic effectiveness, and is suitable for residential, commercial, and healthcare environments.

### SUMMARY OF THE INVENTION

According to a first aspect of the present invention, a phototherapy-integrated showerhead is provided. The showerhead comprises: a showerhead body including a housing defining a water cavity, a water outlet side, and a mounting hole; and a phototherapy unit detachably disposed in the mounting hole, wherein the phototherapy unit is configured to emit therapeutic light outwardly from the water outlet side toward a user during shower operation, thereby enabling simultaneous showering and phototherapy.

In one embodiment of the invention, the housing includes a plurality of water outlet holes communicating with the water cavity, the plurality of water outlet holes being arranged around the mounting hole such that water flow discharged from the water outlet holes surrounds light emitted by the phototherapy unit.

In one embodiment of the invention, the phototherapy unit is detachably connected to the mounting hole through a locking structure selected from a snap-fit connection, a locking protrusion and locking groove engagement, a magnetic attraction structure, thread coupling, or a combination thereof, thereby facilitating installation, removal, and replacement of the phototherapy unit.

In one embodiment of the invention, the phototherapy unit comprises a light-emitting side facing the water outlet side and a backlight side opposite thereto, and includes one or more light-emitting elements configured to emit light of at least one therapeutic wavelength selected from red light, blue light, green light, near-infrared light, or combinations thereof.

In one embodiment of the invention, the showerhead body further comprises a ball joint rotatably connected to the housing and configured for connection with a water supply pipe, allowing adjustment of both a water outlet direction and a light emission direction.

In one embodiment of the invention, the showerhead further comprises a power supply unit, a control unit, and one or more switching elements electrically connected to the phototherapy unit to control activation, light parameters, and operating modes.

In one embodiment of the invention, the phototherapy unit further includes waterproof sealing structures, heat dissipation structures, and hygiene-enhancing features configured to improve safety, durability, and long-term reliability during shower use.

In the context of the specification, when an element is referred to as being "fixed to" or "disposed to" another element, it may either be directly on another element or indirectly on that other element. When a component is said to be "connected" or "connected to" another component, it may be directly connected to another component or indirectly connected to other components on the piece.

In the context of the specification, the terms "first", "second," and "third" are only used for descriptive purposes and do not imply the relative importance or implicitly indicate the quantity of technical features indicated.

In the context of the specification, the term "plurality" means two or more than two, unless otherwise indicated.

In the context of the specification, the term "several" means more than one, unless otherwise specified.

In the context of the specification, the term "beauty device", "therapy device", or "physiotherapy device" refers to the device of the present invention that is configured to perform atomization, phototherapy, thermal therapy, or combined treatment.

In the context of the specification, the term "stimulation element" refers broadly to any component, module, or structure configured to apply a therapeutic or cosmetic stimulus to a user's skin or tissue. Stimulation elements may include, but are not limited to, phototherapy elements, massage elements, microcurrent electrodes, ultrasonic transducers, heating elements, cooling elements, or combinations thereof.

In the context of the specification, the term "phototherapy element" encompasses any light-emitting device capable of emitting light of therapeutic wavelength(s), including but not limited to light-emitting diodes (LEDs), organic LEDs (OLEDs), laser diodes, or equivalent optical sources. The light may include ultraviolet, visible, near-infrared, or farinfrared spectra.

In the context of the specification, the term "housing" is intended to cover any casing, enclosure, or structural body that contains or supports components of the device. The housing may include a handle portion, head portion, or other segments, and may be made from polymeric, metallic, composite, or other suitable materials.

In the context of the specification, the term "light-emitting element," "phototherapy unit," or "light-transmitting surface" refers to a component configured to emit light for skin treatment.

In the context of the specification, the term "mounting part," "mounting groove," or "mounting housing" refers to a structure configured to hold, position, or support the atomizing module.

In the context of the specification, the term "sealing pad," "retaining ring", or "gasket" refers to a component configured to provide a sealed connection between structural elements, preventing liquid or mist leakage.

In the context of the specification, the term "LED module" refers to one or more light-emitting diode (LED) elements that are electrically connected and configured to emit light of specific wavelengths suitable for therapeutic purposes. The LED module may include drive circuitry, heat dissipation structures, and optical elements such as lenses or diffusers to control light distribution.

In the context of the specification, the term "light source" or "phototherapy source" etc. refers to a source emitting coherent laser light, or light-emitting diodes ("LEDs"). The term "light therapy" refers to light generated from any of the sources, such as lasers, LED sources, or Super luminous diodes ("SLD").

In the context of the specification, "Light Emitting Diodes (LEDs)" refer to semiconductor diodes capable of emitting electromagnetic radiation when supplied with an electric current. The LEDs are characterized by superior power efficiencies, smaller sizes, rapid switching speeds, physical robustness, and longer lifespans compared to incandescent or fluorescent lamps. The one or more LEDs may include through-hole type LEDs (generally emitting electromagnetic radiation in red, green, yellow, blue, and white colors), Surface Mount Technology (SMT) LEDs, Bi-color LEDs, Pulse Width Modulated RGB (Red-Green-Blue) LEDs, and high-power LEDs, among others.

Materials used in one or more LEDs may vary from one embodiment to another, depending upon the frequency of radiation required. Different frequencies can be obtained from LEDs made from pure or doped semiconductor materials. Commonly used semiconductor materials include nitrides of Silicon, Gallium, Aluminum, Boron, Zinc Selenide, etc., in pure form or doped with elements such as Aluminum and Indium. For example, red and amber colors are produced from Aluminum Indium Gallium Phosphide (AlGaInP) based compositions, while blue, green, and cyan use Indium Gallium Nitride based compositions. White light may be produced by mixing red, green, and blue lights in equal proportions, while varying proportions may be used to generate a wider color gamut. White and other colored lightings may also be produced using phosphor coatings such as Yttrium Aluminum Garnet (YAG) in combination with a blue LED to generate white light, and Magnesium-doped potassium fluorosilicate in combination with a blue LED to generate red light.

In addition to conventional mineral-based LEDs, one or more LEDs may also be provided on an Organic LED (OLED) based flexible panel or an inorganic LED-based flexible panel. Such OLED panels may be generated by depositing organic semiconducting materials over Thin Film Transistor (TFT) based substrates. Further, a discussion on the generation of OLED panels can be found in Bardsley, J. N. (2004), "International OLED Technology Roadmap", IEEE Journal of Selected Topics in Quantum Electronics, Vol. 10, No. 1, that is included herein in its entirety, by reference. An exemplary description of flexible inorganic light-emitting diode strips can be found in granted U.S. Pat. No. 7,476,557 B2, titled "Roll-to-roll fabricated light sheet and encapsulated semiconductor circuit devices", which is included herein in its entirety by reference.

Unless otherwise stated, the term "light" as used in this specification encompasses electromagnetic radiation in the visible (380-780 nm) and infrared (780 nm-1000 nm) ranges, particularly red light (620-750 nm) and near-infrared (750-1400 nm) wavelengths commonly used in photobiomodulation therapy. Particular wavelengths which may be selected as the dominant emissive wavelength may include the follow, without any preference to be indicated by order: 400 nm, 405 nm, 420 nm, 430 nm, 450 nm, 465 nm, 515 nm, 530 nm, 532 nm, 590 nm, 630 nm, 633 nm, 640 nm, 650 nm, 655 nm, 660 nm, 670 nm, 680 nm, 780 nm, 785 nm, 810 nm, 830 nm, 840 nm, 850 nm, 860 nm, 870 nm, 904 nm, 915 nm, 980 nm, 1015 nm, 1060 nm, 1065 nm, 1070 nm, 1200, and 1400 nm. As used herein, the term "light therapy" refers to the use of one or more light sources of any type that emit light with a wavelength between about 400 and 1400 nm. The device may also emit blue or ultraviolet light for surface-level treatments such as acne reduction or microbial control.

The red light (approximately 630-660 nm) penetrates deeply into the scalp to stimulate blood circulation and enhance hair follicle activity, thus promoting hair growth and repair. Blue light (around 415-470 nm) exhibits antibacterial properties and is effective in treating scalp acne and reducing inflammation. Green light (approximately 520-540 nm) can help reduce pigmentation and soothe sensitive or irritated scalp tissue. Yellow light (around 580-600 nm) improves oxygen exchange in the cells and aids in detoxifying the scalp, while near-infrared light (800-850 nm) reaches deeper layers to accelerate healing and reduce pain.

### BRIEF DESCRIPTION OF THE ACCOMPANYING DRAWINGS

The accompanying drawings illustrate the best mode for carrying out the invention as presently contemplated and set forth hereinafter. The present invention may be more clearly understood from a consideration of the following detailed description of the preferred embodiments taken in conjunction with the accompanying drawings, wherein like reference letters and numerals indicate the corresponding parts in various figures in the accompanying drawings, and in which:
**FIG.** 1 shows a perspective view of a showerhead, in accordance with an embodiment of the present invention.
**FIG.** 2 is an exploded view of a showerhead body and a phototherapy unit of the showerhead, in accordance with an embodiment of the present invention.
FIG. 3 shows a top view of the showerhead, in accordance with an embodiment of the present invention.
FIG. 4 shows a cross-sectional view of the showerhead at section A-A with a point B, in accordance with an embodiment of the present invention.
**FIG.** 5 is an enlarged view of point B in FIG. 4, in accordance with an embodiment of the present invention.

### DETAILED DESCRIPTION

Embodiments of the present invention disclosure will be described more fully hereinafter with reference to the accompanying drawings in which like numerals represent like elements throughout the figures, and in which example embodiments are shown.

The detailed description and the accompanying drawings illustrate the specific exemplary embodiments by which the disclosure may be practiced. These embodiments are described in detail to enable those skilled in the art to practice the invention illustrated in the disclosure. It is to be understood that other embodiments may be utilized, and other changes may be made, without departing from the spirit or scope of the present disclosure. The following detailed description is therefore not to be taken in a limiting sense, and the scope of the present invention disclosure is defined by the appended claims. The claims may, however, be embodied in many different forms and should not be construed as limited to the embodiments set forth herein.

The terms "a" and "an" herein do not denote a limitation of quantity, but rather denote the presence of at least one of the referenced items. The terms "having", "comprising", "including", and variations thereof signify the presence of a component.

Embodiments of the present invention disclose a showerhead incorporating a phototherapy unit, wherein the phototherapy unit is structurally integrated with a showerhead body and configured to emit therapeutic light toward a user during shower operation. In the embodiments described herein, the phototherapy unit is detachably or removably mounted within the mounting hole, which is located at the center of the showerhead body, allowing the showerhead to simultaneously perform conventional water delivery and phototherapy functions. Through this integrated configuration, the invention enriches the functional capabilities of a traditional showerhead while maintaining a compact structure and ease of use.

In an embodiment, the showerhead body includes a housing defining a water cavity, a water outlet side, and a mounting hole configured to receive the phototherapy unit. The phototherapy unit may be secured within the mounting hole using one or more detachable connection structures, such as snap-fit mechanisms, locking protrusion-and-groove structures, magnetic coupling arrangements, thread coupling, or combinations thereof. These structural arrangements enable reliable fixation during use while allowing convenient installation, removal, replacement, or upgrading of the phototherapy unit as needed.

In an embodiment, the phototherapy unit includes one or more light-emitting elements configured to emit therapeutic light of one or more wavelengths, such as red light, blue light, near-infrared light, or combinations thereof. The phototherapy unit may further include optical structures, control units, power supply units, and safety features that cooperate to deliver controlled and uniform phototherapy during showering.

Additionally, the present invention contemplates multiple optional embodiments relating to power supply modes, control and switching mechanisms, optical light distribution structures, smart and wireless functions, user interface and indication features, waterproofing and sealing arrangements, and hygiene and maintenance enhancements. These embodiments may be implemented individually or in any suitable combination, depending on application requirements, without departing from the scope of the present invention.

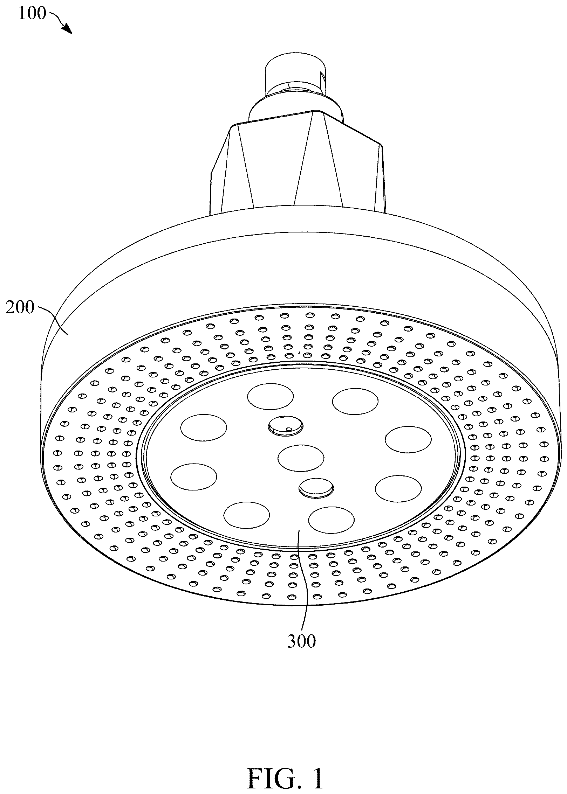

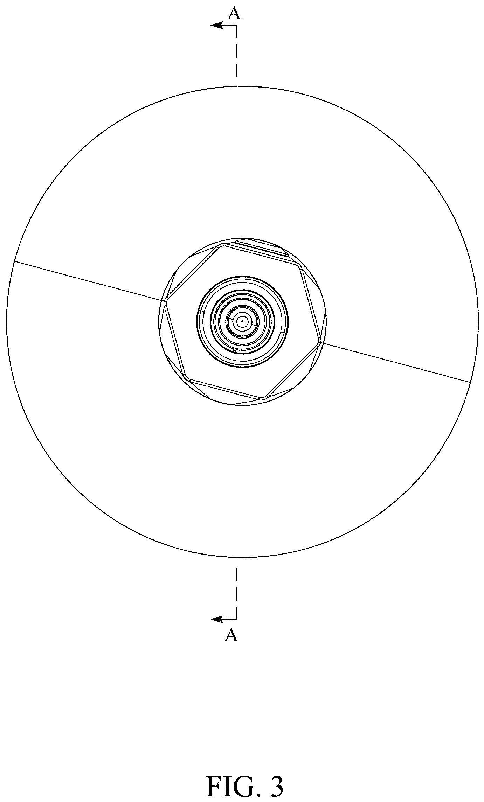

Referring to **FIGS. 1** to **5****,** a showerhead 100 is provided. The showerhead 100 comprises a showerhead body 200 and a phototherapy unit 300. The showerhead body 200 includes a housing 202, which defines a mounting hole 210 formed therein. The phototherapy unit 300 is detachably disposed within the mounting hole 210. The phototherapy unit 300 is configured to emit therapeutic light outwards from the mounting hole 210 toward a user, thereby performing phototherapy on the human body during shower operation.

The showerhead body 200 and the phototherapy unit 300 together are configured to deliver water for bathing while simultaneously providing phototherapy to a user, thereby achieving a multi-functional bathing device.

The showerhead body 200 forms a main structural portion of the showerhead 100 and is configured to connect to a water supply and discharge water toward a user. The showerhead body 200 includes the housing 202, which defines the overall external shape of the showerhead 100 and supports various functional components. The housing 202 is configured to accommodate both the water flow components and the phototherapy unit 300 in an integrated manner.

By incorporating the phototherapy unit 300 into the showerhead 100, the showerhead 100 is capable of simultaneously performing both conventional showering functions and phototherapy functions, thereby significantly enriching the functional characteristics of the showerhead 100. Furthermore, since the phototherapy unit 300 is detachably connected to the mounting hole 210, installation, removal, maintenance, and replacement of the phototherapy unit 300 are facilitated. In this manner, when the phototherapy unit 300 malfunctions, reaches the end of its service life, or requires replacement, it can be conveniently removed and replaced without replacing the entire showerhead 100.

In an embodiment, the phototherapy unit 300 is configured to emit light of one or more predetermined wavelengths, such as red light, blue light, or other therapeutic wavelengths, to achieve different phototherapy effects corresponding to skin care, relaxation, or other wellness-related treatments.

In an embodiment, the phototherapy unit 300 is detachably engaged with the wall of the mounting hole 210 by a snap-fit connection. The snap-fit configuration is structurally simple and does not require additional fastening tools or complex assembly operations, thereby enabling convenient installation and disassembly of the phototherapy unit 300. Additionally, the snap-fit connection provides reliable positional stability, ensuring that the phototherapy unit 300 remains securely retained within the mounting hole 210 during use, and reducing the likelihood of loosening, displacement, or detachment caused by water flow impact or external forces.

It should be noted that the manner of attachment of the phototherapy unit 300 is not limited to the snap-fit configuration described above. In an embodiment, the phototherapy unit 300 may be magnetically coupled to the showerhead body 200, such that the phototherapy unit 300 is magnetically attracted to the mounting hole 210. The magnetic attachment configuration likewise enables convenient installation and removal while providing sufficient retention stability during normal operation. In some embodiment, the phototherapy unit 200 may be coupled using threads to the shower head body 200.

In an embodiment, the wall of the mounting hole 210 is provided with at least one locking protrusion 212, and the outer peripheral side 306 of the phototherapy unit 300 is correspondingly provided with at least one locking groove 308, such that the locking protrusion 212 is configured to engage with the locking groove 308 to achieve a detachable locking connection.

Furthermore, in an embodiment, the outer peripheral side 306 of the phototherapy unit 300 may be provided with the locking protrusion 212, while the wall of the mounting hole 210 is provided with the locking groove 308, thereby realizing the same locking and positioning effect through an inverted structural arrangement.

In an embodiment, along a depth direction of the mounting hole 210, the phototherapy unit 300 includes a light-emitting side 302 and a backlight side 304 disposed opposite to each other. The locking groove 308 is configured as a composite groove structure and includes a sliding groove section 310, a connecting groove section 312, and a locking groove section 314 that are sequentially connected. The sliding groove section 310 and the locking groove section 314 each extend substantially along the depth direction of the mounting hole 210, while the connecting groove section 312 extends circumferentially along the inner wall of the mounting hole 210. One end of the sliding groove section 310 is located at the backlight side 304 of the phototherapy unit 300, and the other end thereof is positioned between the light-emitting side 302 and the backlight side 304. The connecting groove section 312 and the locking groove section 314 are likewise located between the light-emitting side 302 and the backlight side 304, wherein one end of the connecting groove section 312 is connected to the end of the sliding groove section 310 adjacent the light-emitting side 302, and the other end of the connecting groove section 312 is connected to the end of the locking groove section 314 adjacent the light-emitting side 302.

During installation of the phototherapy unit 300, the locking protrusion 212 is guided into the sliding groove section 310 from the end located at the backlight side 304, then moved along the connecting groove section 312, and finally engaged within the locking groove section 314. The locking groove section 314 restricts the movement of the locking protrusion 212, thereby securely retaining the phototherapy unit 300 within the mounting hole 210. During disassembly, the locking protrusion 212 is moved in a reverse direction from the locking groove section 314, through the connecting groove section 312, and back into the sliding groove section 310, allowing the phototherapy unit 300 to be removed.

Through the cooperation of the groove and the protrusion, the installation and removal of the phototherapy unit 300 are smooth and intuitive, while a stable and reliable connection is maintained after installation. This structure effectively reduces the likelihood of loosening or detachment of the phototherapy unit 300 caused by water flow impact, vibration, or other external forces during use.

It should be noted that the configuration of the locking groove 308 is not limited to the foregoing composite groove structure. In other embodiments, the locking groove 308 may be configured as a V-shaped groove, wherein the sliding groove section 310 and the locking groove section 314 are connected at an angle, and one end of the sliding groove section 310 is located on the backlight side 304 of the phototherapy unit 300, thereby similarly enabling guided insertion and secure locking of the phototherapy unit 300 within the mounting hole 210.

In an embodiment, a plurality of locking protrusions 212 and a plurality of locking grooves 308 are provided, wherein each locking protrusion 212 corresponds to a respective locking groove 308. The plurality of locking protrusions 212 is arranged sequentially and spaced apart along a circumferential direction of the mounting hole 210. Through cooperative engagement between the plurality of locking protrusions 212 and the plurality of locking grooves 308, and by virtue of their circumferential distribution, a more uniform and balanced connection is achieved between the phototherapy unit 300 and the mounting hole 210. As a result, forces acting on the phototherapy unit 300 during use are effectively dispersed, thereby further enhancing the stability and reliability of the connection between the phototherapy unit 300 and the mounting hole 210.

In an embodiment, along the depth direction of the mounting hole 210, the phototherapy unit 300 includes a light-emitting side 302 and a backlight side 304 disposed opposite to each other, and the outer diameter of the phototherapy unit 300 gradually decreases from the light-emitting side 302 toward the backlight side 304. This tapered structural configuration facilitates guided insertion of the phototherapy unit 300 into the mounting hole 210, reduces alignment difficulty during installation, and improves overall installation convenience and efficiency.

The mounting hole 210 may be formed in various structural configurations. In some embodiments, the mounting hole 210 is configured as a blind hole. The blind hole structure provides protective accommodation for the backlight side 304 of the phototherapy unit 300, thereby reducing exposure to external environmental factors and extending the service life of the phototherapy unit 300. However, the present disclosure is not limited thereto, and in other embodiments, the mounting hole 210 may alternatively be configured as a through hole.

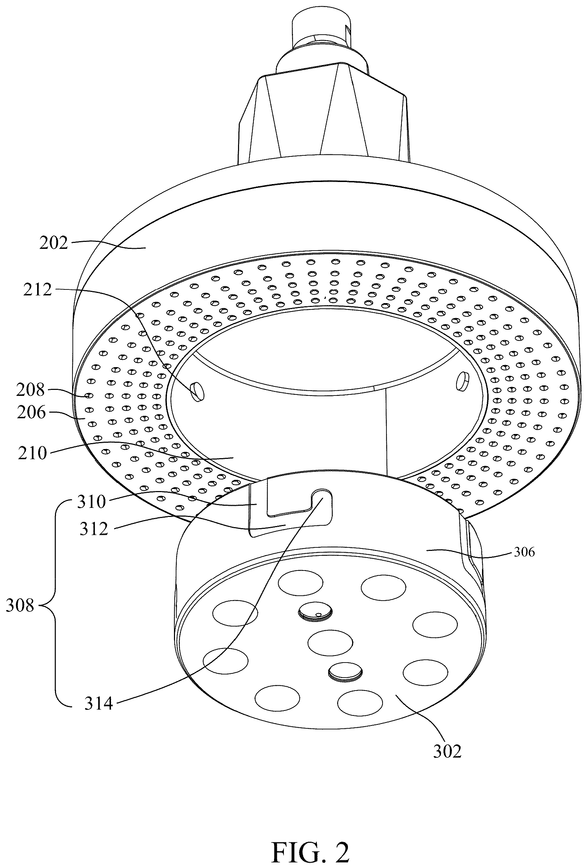

Referring to **FIG. 2****,** the housing 202 of the showerhead body 200 includes a water outlet side 206, from which water is discharged during use. A plurality of water outlet holes 208 are formed on the water outlet side 206 and are configured to spray water outwardly toward the user.

The housing 202 further defines a mounting hole 210 formed on the water outlet side 206. The mounting hole 210 is configured to receive the phototherapy unit 300, and the plurality of water outlet holes 208 are arranged around the mounting hole 210, such that water discharged from the water outlet holes 208 surrounds light emitted by the phototherapy unit 300 during operation. This structural arrangement allows water flow and therapeutic light to act simultaneously on the user's body.

In some embodiments, the housing 202 is provided with one or more locking protrusions 212 formed on an inner wall of the mounting hole 210. The locking protrusions 212 are configured to cooperate with corresponding slot structures of the phototherapy unit 300 to detachably secure the phototherapy unit 300 within the mounting hole 210.

The phototherapy unit 300 includes a light-emitting side 302 and an outer peripheral side 306. The light-emitting side 302 is oriented toward the water outlet side 206 when the phototherapy unit 300 is installed in the mounting hole 210, and is configured to emit therapeutic light outwardly toward the user. The water outlet side 206 can be made of silicone to protect the internal elements of the shower head 100. The outer peripheral side 306 is configured to face the inner wall of the mounting hole 210 and to cooperate with the locking protrusions 212 for secure installation.

In some embodiments, the outer peripheral side 306 of the phototherapy unit 300 is provided with a locking groove 308 configured to receive the locking protrusion 212. The locking groove 308 may include a sliding groove section 310, a connecting groove section 312, and a locking groove section 314 connected in sequence. The sliding groove section 310 allows initial insertion of the locking protrusion 212 during installation, the connecting groove section 312 guides circumferential movement of the phototherapy unit 300, and the locking groove section 314 retains the locking protrusion 212 to stably secure the phototherapy unit 300 in the mounted position.

Through the cooperation between the locking protrusion 212 and the locking groove 308, the phototherapy unit 300 can be conveniently installed, removed, or replaced while maintaining stable fixation during use. This detachable connection structure improves ease of maintenance and enhances the reliability of the showerhead 100 with integrated phototherapy unit.

In an embodiment, the LED phototherapy unit 300 is detachably coupled to the shower head body, allowing the unit 300 to be removed independently for charging. Electrical isolation between the LED phototherapy unit 300 and the shower head body is achieved when the unit 300 is detached, thereby enabling safe charging away from water exposure. This detachable configuration improves user safety and maintenance convenience.

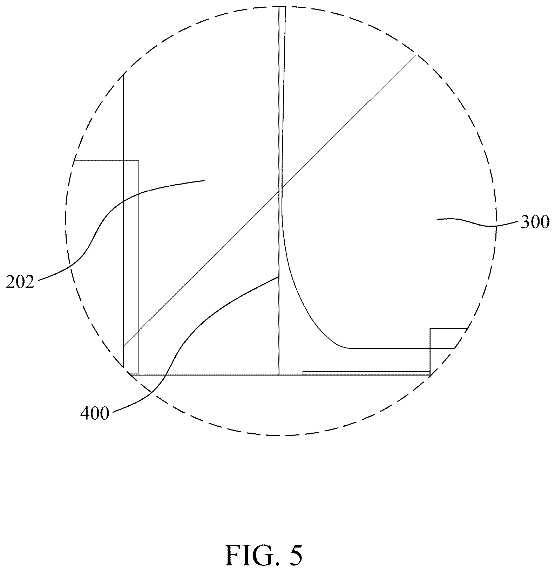

Referring to **FIGS. 2** and **5****,** in an embodiment, an exhaust gap 400 is defined between the outer peripheral side 306 of the phototherapy unit 300 and an inner wall of the mounting hole 210, with the exhaust gap 400 being in fluid communication with an external environment outside the mounting hole 210. During installation of the phototherapy unit 300, air within the mounting hole 210 is discharged through the exhaust gap 400, thereby preventing air pressure resistance and improving the smoothness of the installation process. Additionally, the exhaust gap 400 facilitates heat dissipation by allowing heat generated by the phototherapy unit 300 during operation to be released to the external environment, thereby reducing heat accumulation and minimizing adverse effects on the performance and service life of the phototherapy unit 300.

In some embodiments, the housing 202 defines a water cavity 204 and a water outlet side 206. A plurality of water outlet holes 208 are formed in the water outlet side 206 and are in fluid communication with the water cavity 204. The mounting hole 210 is formed on the water outlet side 206, and the plurality of water outlet holes 208 are circumferentially arranged around the mounting hole 210. With this configuration, therapeutic light emitted by the phototherapy unit 300 is directed outwardly from the water outlet side 206 toward the user. Simultaneously, water sprayed from the plurality of water outlet holes 208 surrounds the emitted light, allowing the light to be uniformly applied to body regions being rinsed by the water flow during showering. This arrangement enhances integration between the phototherapy effect and the showering process and improves user experience. Additionally, this arrangement makes efficient use of the space on the water outlet side 206, thereby maintaining compactness of the overall structure of the showerhead 100.

In some embodiments, the housing 202 may have a dome-shaped, bell-shaped, or hemispherical profile. This configuration may provide an aesthetically pleasing appearance while accommodating the internal water cavity 204 and supporting the arrangement of water outlet holes 208 and the mounting hole 210 on the water outlet side 206. The curved profile of the housing 202 may also facilitate water flow distribution within the water cavity 204 and improve the structural rigidity of the showerhead body 200.

In some embodiments, the plurality of water outlet holes 208 may be arranged in concentric rings or circular patterns around the mounting hole 210. The mounting hole 210 may be centrally located on the water outlet side 206, such that the water outlet holes 208 are distributed symmetrically around the phototherapy unit 300. This arrangement may provide uniform water distribution during showering while allowing therapeutic light emitted from the centrally positioned phototherapy unit 300 to reach the user simultaneously with the surrounding water flow.

In an embodiment of the present invention, the outer peripheral side 306 and the inner wall of the mounting hole 210 are sealed together by contacting surface to surface to surface to form a closed space. The closed space is formed by sealing the exhaust gap. The heat generated by the phototherapy unit is dissipated through the inner wall of the housing 202 or inner wall of the mounting hole 210, into the water cavity 204, which can then be used to heat the water flowing in the shower head.

In some embodiments, the light-emitting side 302 of the phototherapy unit 300 may include a plurality of apertures, openings, or light-transmitting windows through which therapeutic light is emitted toward the user. The apertures may be circular, rectangular, or other suitable shapes, and may be arranged in a pattern configured to provide desired light distribution characteristics. A transparent or translucent cover may be disposed over the apertures to protect internal light-emitting elements while permitting transmission of therapeutic light.

In some embodiments, the phototherapy unit 300 may have a polygonal outer profile on the outer peripheral side 306. For example, the outer peripheral side 306 may have a hexagonal, octagonal, or other non-circular cross-sectional shape. The polygonal outer profile may cooperate with a correspondingly shaped inner wall of the mounting hole 210 to provide rotational alignment or anti-rotation engagement between the phototherapy unit 300 and the showerhead body 200. This configuration may ensure that the phototherapy unit 300 is installed in a predetermined angular orientation and may prevent unintended rotation during use.

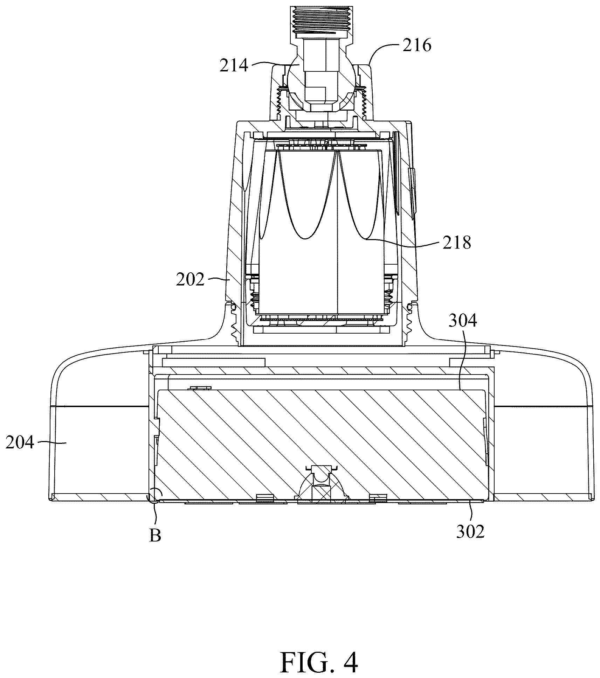

Referring to **FIG. 4****,** in an embodiment, the showerhead body 200 further includes a ball joint 214 rotatably mounted to the housing 202 and configured for connection with a water supply pipe. The ball joint 214 enables the housing 202 to rotate relative to the water supply pipe across multiple angular orientations, thereby allowing a user to conveniently adjust both the water discharge direction of the showerhead 100 and the light emission direction of the phototherapy unit 300 according to usage requirements.

The rotatable connection provided by the ball joint 214 ensures stable mechanical support of the showerhead body 200 while permitting smooth and continuous adjustment during operation. The ball joint 214 further maintains reliable water flow communication between the water supply pipe and the housing 202 during rotational movement, thereby preventing leakage or flow interruption and enhancing overall operational stability and user experience.

In an embodiment, the shower head assembly is configured to maintain waterproof performance when operated in a predefined orientation. The LED phototherapy unit 300 and associated electrical components are sealed such that, when the LED light-emitting surface is oriented downward during normal shower use, the device achieves a predetermined ingress protection rating. This orientation-dependent sealing structure enhances water resistance during operation.

In some embodiments, the ball joint 214 may include a threaded fitting configured for secure connection to the water supply pipe. The threaded fitting may be an internally threaded or externally threaded connector compatible with standard plumbing fittings. The ball joint 214 may further include one or more internal biasing elements, such as springs or resilient members, configured to provide rotational resistance or positional stability. The biasing elements may apply a frictional or spring-loaded force that maintains the housing 202 in a selected angular position while still permitting manual adjustment by the user. This configuration may prevent unintended movement of the showerhead 100 during use while allowing convenient repositioning when desired.

In one embodiment, the showerhead body 200 comprises a water inlet side 216, and the water inlet side 216 is integrated in the housing 202. The housing 202 is detachably connected with the shower head body 200 and has a water inlet flow path wherein a filter 218 is disposed. The filter 218 is positioned along a water inlet flow path such that incoming water passes through the filter 218 before reaching the water outlet side 206. This arrangement enables the filter 218 to perform both filtration and flow-buffering functions, thereby reducing impurities in the incoming water while promoting a more stable and uniform water output during use. In an optional configuration, the water inlet side 216 and the water outlet side 206 are thread-connected, snap-connected, or otherwise removably coupled, which enhances sealing performance at the joint and reduces the risk of water leakage during operation.

In one embodiment, the filter 218 comprises a plurality of functional filtration layers arranged sequentially along the water flow direction. The filtration layers may include a blocking layer formed by a stainless-steel filter screen configured to intercept silt, rust, and relatively large particulate impurities present in the incoming water. Downstream of the blocking layer, a high-precision polypropylene cotton filter layer may be provided to remove suspended solids and fine particulate matter. These initial filtration layers cooperate to reduce particulate load and protect downstream filtration materials from premature degradation.

In one embodiment, the filter 218 further comprises one or more conditioning and adjustment layers configured to improve water quality. Such layers may include an alkaline sphere adjustment layer configured to regulate water pH, release trace minerals, and generate negative ions, thereby improving the perceived quality of the water. Additionally, a bactericidal sterilization layer comprising KDF-based filtration media may be provided to remove residual chlorine, reduce heavy metal content, and inhibit microbial growth within the water flow path. These layers collectively contribute to chemical stabilization and antimicrobial conditioning of the water.

In one embodiment, the filter 218 further comprises improvement and adsorption layers configured to enhance water purity and sensory characteristics. A ceramic ball improvement layer may be provided to reduce residual chlorine while retaining beneficial minerals and improving water taste. An activated carbon adsorption layer may be arranged downstream to adsorb residual chlorine, odors, discoloration, and organic contaminants. In addition, a calcium sulfite layer may be included to rapidly neutralize chlorine, chlorinated by-products, and rust particles, particularly under conditions of high water flow or elevated temperature.

In one embodiment, the filter 218 further comprises a mineralized stone layer configured to release beneficial trace elements into the water during filtration. The mineralized stone layer may include medical stone or maifanite material that gradually mineralizes the water while maintaining stable filtration performance. Collectively, the plurality of filtration layers cooperate to purify, mineralize, sterilize, and condition water delivered through the showerhead, thereby enhancing user comfort and supporting prolonged use of the showerhead in a domestic environment.

In an embodiment, an upper portion of the shower head body 200 is detachably connected to allow access to an internal filter 218. The filter 218 may be removed and replaced without disassembling the entire showerhead 100 . This structure facilitates routine maintenance and ensures sustained water filtration performance over the operational lifetime of the device.

In some embodiments, the phototherapy unit 300 includes one or more waterproof sealing structures configured to prevent water ingress into internal electrical components during shower operation. The waterproof sealing structures may include O-rings, gaskets, or sealing rings positioned between the outer peripheral side 306 of the phototherapy unit 300 and the inner wall of the mounting hole 210. In some cases, the sealing structures may be formed from elastomeric materials such as silicone rubber, EPDM rubber, or fluorocarbon rubber, which provide reliable sealing performance under repeated exposure to water, humidity, and temperature variations. Additionally, internal circuit components may be protected using potting compounds, conformal coatings, or encapsulation materials that form a moisture-resistant barrier around sensitive electronic elements. The phototherapy unit 300 may be configured to meet or exceed an ingress protection rating such as IP65, IP67, or IP68, depending on application requirements and expected exposure conditions.

In some embodiments, the phototherapy unit 300 is configured to operate at low voltage levels to enhance electrical safety during use in wet environments. The operating voltage may be in a range of approximately 3.3V to 12V DC, which reduces the risk of electrical shock in the event of water contact with electrical components. The rechargeable battery and control circuitry may be electrically isolated from water-exposed surfaces through the use of insulating housings, potting compounds, or encapsulation layers. Circuit boards within the phototherapy unit 300 may be coated with conformal coatings, such as acrylic, silicone, or urethane-based coatings, to provide an additional layer of protection against moisture and corrosion. Insulating materials, such as engineering plastics or ceramic substrates, may be used to separate conductive elements from external surfaces of the phototherapy unit 300.

In some embodiments, the phototherapy unit 300 includes an internal assembly comprising a circuit board, such as a printed circuit board (PCB), configured to support one or more light-emitting elements and associated driver circuitry. The light-emitting elements, which may include LEDs or other suitable light sources, may be mounted on a surface of the circuit board facing the light-emitting side 302 of the phototherapy unit 300. The circuit board may further support control circuitry, power management components, and electrical connectors for coupling to the rechargeable battery or external power source. A protective cover or lens may be disposed over the light-emitting elements to protect them from mechanical damage and water exposure while permitting transmission of therapeutic light. The internal components may be housed within a body portion of the phototherapy unit 300, which may be formed from polymeric or composite materials and configured to provide structural support and environmental protection.

In some embodiments, the phototherapy unit 300 includes one or more heat dissipation structures configured to manage thermal energy generated by the light-emitting elements during operation. The heat dissipation structures may include heat sinks formed from thermally conductive materials such as aluminum or copper, which are thermally coupled to the light-emitting elements or the circuit board. In some cases, the circuit board may comprise a metal-core PCB (MCPCB) or an insulated metal substrate (IMS) that provides enhanced thermal conductivity compared to standard FR-4 substrates. Thermal interface materials, such as thermal pads, thermal paste, or thermally conductive adhesives, may be disposed between heat-generating components and heat dissipation structures to improve thermal transfer efficiency. The exhaust gap 400 between the phototherapy unit 300 and the mounting hole 210 may cooperate with internal heat dissipation structures to facilitate convective heat transfer to the surrounding environment, thereby maintaining operating temperatures within acceptable limits and prolonging component service life.

In some embodiments, the phototherapy unit 300 is configured with optical design parameters selected to provide effective therapeutic light distribution. The light-emitting elements may be configured with beam angles in a range of approximately 30 degrees to 120 degrees, depending on the desired coverage area and light intensity distribution. Diffuser elements, such as frosted lenses or textured optical surfaces, may be disposed over the light-emitting elements to promote uniform light distribution across the illuminated area and reduce localized intensity variations. A protective transparent cover may be disposed on the light-emitting side 302 of the phototherapy unit 300 and may be formed from optically transparent materials such as polycarbonate, acrylic (PMMA), or glass, which provide mechanical protection while maintaining high optical transmittance. The protective cover may be sealed to the body of the phototherapy unit 300 using adhesive bonding, ultrasonic welding, or mechanical fastening with intervening gaskets to maintain waterproof integrity.

In an embodiment, the phototherapy unit 300 comprises an internal rechargeable battery configured to supply electrical power to one or more light-emitting elements disposed within the phototherapy unit 300. The rechargeable battery may be selected from standard alkaline batteries, lithium-ion batteries, lithium-polymer batteries, nickel-metal hydride batteries, or other suitable rechargeable energy storage devices. The rechargeable battery is disposed within an internal cavity of the phototherapy unit 300 or within the housing 202 and is electrically connected to a control circuit that manages charging, discharging, and power regulation. The internal cavity is sealed using waterproof and moisture-resistant structures to prevent water ingress during shower operation.

In an embodiment, the rechargeable batteries can be multiple cylindrical battery cells arranged to provide sufficient power for the extended operation of the LED phototherapy unit 300. The rechargeable battery is configured to supply electrical power for a cumulative operating duration of at least approximately 200 minutes between charging cycles. The rechargeable battery may further be selected to comply with recognized battery safety and transportation standards.

In an embodiment, the rechargeable battery is configured to be charged through a wired charging interface. The wired charging interface may comprise a USB interface, such as a USB Type-C interface, a micro-USB interface, or another standardized low-voltage charging interface. The charging interface may be disposed on the outer surface of the phototherapy unit 300 or the housing 202 and can be protected by a waterproof cover, sealing plug, or magnetic waterproof connector. Through this configuration, the rechargeable battery can be conveniently charged using common charging devices, such as mobile phone chargers or power adapters, thereby improving user convenience and compatibility with existing charging infrastructure.

In an embodiment, the rechargeable battery is configured to be charged wirelessly. The phototherapy unit 300 may include a wireless charging receiving module configured to receive electrical energy through electromagnetic induction or magnetic resonance. The wireless charging receiving module may be disposed within the phototherapy unit 300 and electrically coupled to the rechargeable battery. In this embodiment, the phototherapy unit 300 or the showerhead 100 may be placed on or near a corresponding wireless charging base when not in use, allowing the rechargeable battery to be charged without physical electrical contacts. This configuration further improves waterproof performance, reduces wear of charging ports, and enhances long-term reliability.

In an embodiment, the phototherapy unit 300 further includes a charging management circuit configured to prevent overcharging, over-discharging, overheating, or short-circuit conditions of the rechargeable battery. The charging management circuit may cooperate with temperature sensors or voltage detection units to ensure safe and stable charging operation, thereby extending the service life of the rechargeable battery and improving the overall safety of the showerhead 100.

In an embodiment, the phototherapy unit 300 comprises a control unit configured to selectively activate and deactivate one or more light-emitting elements disposed within the phototherapy unit 300. The control unit may include a manually operable switch disposed on an outer surface of the phototherapy unit 300 or the housing 202. The manually operable switch may be configured as a mechanical push button, sliding switch, rotary switch, or other suitable actuation structure capable of withstanding a wet operating environment.

In an embodiment, the control unit includes a touch-based switching structure. The touch-based switching structure may be a capacitive touch sensor, a resistive touch sensor, or a pressure-sensitive touch sensor disposed beneath a waterproof outer layer of the phototherapy unit 300. When a user touches or presses a predetermined area, the control unit detects the input signal and activates or deactivates the light-emitting elements accordingly. This configuration eliminates mechanical moving parts, reduces wear, and improves long-term reliability while maintaining ease of operation.

In an embodiment, the control unit is further configured to respond to different user inputs, such as short presses, long presses, or multiple sequential touches, to perform different control functions. For example, a short press may be configured to turn the phototherapy unit 300 on or off, while a long press may be configured to switch between different light wavelengths or intensity levels. This multi-function control approach improves user convenience without increasing the number of physical controls.

In an embodiment, the control unit is configured to selectively adjust operating parameters of the phototherapy unit 300. The adjustable parameters may include light wavelength, light intensity, emission duration, emission frequency, or emission pattern. For example, the phototherapy unit 300 may be configured to emit red light, blue light, near-infrared light, or combinations thereof, depending on a selected operating mode.

In one embodiment, the showerhead 100 comprises a built-in LED phototherapy unit 300 including a plurality of light-emitting diodes configured to emit light at two different wavelengths. The phototherapy unit 300 includes multiple LEDs emitting light at approximately 650 nm and multiple LEDs emitting light at approximately 850 nm, thereby providing dual-wavelength phototherapy. The LEDs may be arranged such that light at both wavelengths is emitted simultaneously toward a treatment region during operation.

In an embodiment, the control unit enables users to cycle through different phototherapy modes via repeated actuation of the control interface. Each mode may correspond to a predetermined set of light parameters optimized for specific therapeutic purposes, such as skin care, relaxation, or muscle stimulation. The control unit may store these predefined modes in an internal memory.

In an embodiment, the control unit is further configured to dynamically adjust light output based on operating conditions. For example, the control unit may reduce light intensity when water temperature exceeds a predetermined threshold or when prolonged operation is detected, thereby improving user safety and component longevity.

In an embodiment, the phototherapy unit 300 includes a timing control unit configured to limit the duration of light emission. The timing control unit may automatically deactivate/shut off the light-emitting elements after a predetermined operating time has elapsed. The predetermined time may be fixed or user-adjustable and may be selected to correspond to recommended phototherapy durations.

In an embodiment, the phototherapy unit 300 is configured to terminate operation upon completion of a predetermined treatment session automatically. The phototherapy unit 300 may further include a feedback mechanism configured to notify the user that the treatment session has ended. Such feedback may be provided through visual indicators, audible signals, or a combination thereof, thereby improving user awareness and preventing unintended prolonged operation.

In an embodiment, the LED phototherapy unit 300 comprises an integrated audible notification device, such as a buzzer or speaker. The audible notification device is configured to emit an audible signal upon at least one of powering ON the device, powering OFF the device, completion of a treatment session, or detection of a low-battery condition. The audible notification enhances user interaction by providing non-visual operational feedback during use in wet or low-visibility environments.

In an embodiment, the timing control unit is configured to operate independently of water flow detection. For example, even if water continues to flow, the phototherapy unit 300 may be automatically deactivated after the predetermined duration, thereby preventing excessive exposure to therapeutic light and reducing unnecessary power consumption.

In an embodiment, the LED phototherapy unit 300 is configured to operate in a plurality of selectable treatment durations. The treatment duration may be pre-set or selectively adjustable by a user, including but not limited to durations of approximately 5 minutes, 10 minutes, or 15 minutes. Upon selecting a desired treatment duration, the LED phototherapy unit 300 automatically operates for the specified time period and subsequently shuts down without further user intervention. This configuration ensures consistent and repeatable treatment sessions.

In an embodiment, the timing control unit cooperates with other control functions, such as mode selection or flow detection, to provide layered safety protection. This multilevel control strategy improves operational reliability and ensures safe and effective use of the phototherapy unit 300.

In an embodiment, the phototherapy unit 300 integrates both manual control and automatic control functions. For example, a user may manually enable the phototherapy unit 300, while actual activation and deactivation are governed by water-flow detection or timing control. Alternatively, the phototherapy unit 300 may be automatically activated by water flow but manually overridden by user input.

This hybrid control configuration provides greater flexibility and allows users to customize operation according to personal preferences. At the same time, automatic control functions maintain safety, energy efficiency, and synchronization with shower operation.

In an embodiment, the shower head system further comprises a remote control configured to wirelessly communicate with the LED phototherapy unit 300. The remote control includes a plurality of user-operable buttons and a display unit configured to present operational parameters of the device. The remote control enables a user to remotely power the LED phototherapy unit 300 ON or OFF and to control treatment parameters without physically accessing the shower head. Wireless communication between the remote control and the LED phototherapy unit 300 may be established using Bluetooth, radio frequency, or other short-range wireless communication protocols.

In an embodiment, the phototherapy unit 300 includes one or more optical lens structures configured to shape, focus, or redirect emitted light. The lens structures may be disposed on the light-emitting side 302 or within the interior of the phototherapy unit 300 and may include convex lenses, concave lenses, Fresnel lenses, or combinations thereof. The lens structures may be integrally formed with a protective cover plate or provided as separate optical components.

In an embodiment, the phototherapy unit 300 includes a light-guiding structure, such as a light-guiding ring or optical waveguide, configured to guide light emitted by the light-emitting elements along a predetermined path. The light-guiding structure may distribute light circumferentially around the mounting hole 210, thereby forming a ringshaped or annular light emission pattern surrounding the water outlet region.

Using lenses and light-guiding structures, emitted light can be directed toward target areas of the human body with improved precision and efficiency. This configuration increases utilization of emitted light, reduces optical losses, and enhances consistency of therapeutic coverage during shower operation.

In an embodiment, the phototherapy unit 300 further includes one or more reflective structures disposed within the interior of the phototherapy unit 300. The reflective structures may be formed from reflective coatings, metallic films, or reflective polymer layers applied to internal surfaces of the phototherapy unit 300.

The reflective structures are configured to reflect light emitted by the light-emitting elements toward the light-emitting side 302, thereby reducing backward or lateral light loss. This configuration improves optical efficiency and ensures that a greater proportion of emitted light is directed toward the user.

In an embodiment, the reflective structures are selectively arranged to shape the light emission profile. For example, reflective surfaces may be angled or curved to redirect light toward specific regions, thereby further optimizing therapeutic effectiveness without increasing power consumption.

In an embodiment, the phototherapy unit 300 includes an optical adjustment mechanism configured to modify light intensity or distribution characteristics. The optical adjustment mechanism may include adjustable lenses, movable diffusers, or electronically controlled light modulation elements.

In an embodiment, the LED phototherapy unit 300 is configured to emit therapeutic light within predefined output ranges at varying distances from a treatment surface. The emitted light intensity may be selected or regulated such that the combined output of the LEDs delivers effective phototherapy within a predetermined distance range from the user's body. The light output may be calibrated to maintain therapeutic effectiveness while minimizing excessive exposure.

In an embodiment, the LED phototherapy unit 300 is configured for use at a predetermined distance from a user's body. The device may be optimized to deliver therapeutic light when positioned at a distance range between approximately 10 cm and 20 cm from the skin surface. This distance-based configuration enhances safety and ensures consistent therapeutic performance during normal shower use.

In an embodiment, the control unit cooperates with the optical adjustment mechanism to automatically adjust light distribution based on selected operating modes or environmental conditions. For example, a wider light distribution may be selected for general wellness applications, while a more focused distribution may be selected for targeted therapeutic use.

This adjustable optical configuration enhances the versatility of the phototherapy unit 300 and allows the showerhead 100 to accommodate a variety of phototherapy applications without structural modification.

In an embodiment, the phototherapy unit 300 includes a smart control unit configured to store operational data and user preferences. The smart control unit may include memory configured to store data such as usage frequency, operating duration, selected light wavelengths, or historical therapy modes. This stored data may be accessed locally or transmitted wirelessly to an external device for monitoring or analysis.

In an embodiment, the smart control unit is configured to automatically adjust operation based on stored data or predefined rules. For example, the phototherapy unit 300 may automatically activate a preferred phototherapy mode when a particular user profile is selected or may adjust light intensity based on cumulative usage time. This intelligent control capability improves personalization, consistency of therapy, and overall user experience.

In an embodiment, the phototherapy unit 300 includes a visual indication unit configured to provide real-time feedback regarding operational status. The visual indication unit may include one or more indicator lights, light rings, display elements, or illuminated symbols disposed on the phototherapy unit 300 or the housing 202.

In an embodiment, the LED phototherapy unit 300 comprises at least two indicator lights configured to display battery status information. The indicator lights may operate in different illumination patterns to represent varying battery charge levels. For example, both indicator lights may remain continuously illuminated to indicate a high battery charge, a single indicator light may remain illuminated to indicate a reduced charge level, and one or more indicator lights may flash at approximately 1 Hz to indicate a low-battery condition. Such visual feedback allows the user to monitor battery status during use.

In an embodiment, the indicator lights are further configured to provide visual feedback during charging of the LED phototherapy unit 300. During charging, the indicator lights may illuminate or flash in a predefined sequence to indicate that charging is in progress. Upon completion of charging, the indicator lights may change to a steady illumination state to indicate that the battery system is fully charged. This embodiment enables intuitive monitoring of the charging state.

In an embodiment, the phototherapy unit 300 is activated by pressing and holding a power button for approximately one second, upon which an indicator light illuminates and the LEDs in the treatment area are energized for a default treatment duration of about 10 minutes. When the device is not activated, it remains in a standby state and may be powered ON or OFF using a remote control.

In an embodiment, the showerhead 100 is configured to achieve an IPX7 waterproof rating during normal use when the phototherapy unit 300 is oriented facing downward, thereby ensuring reliable operation in a wet shower environment.

In an embodiment, the showerhead 100 has an overall size of approximately 170 mm × 145 mm, allowing for compact integration of the phototherapy unit 300, filter 218, and water outlet components (208 and 206) while maintaining ergonomic usability.

The visual indication unit may be configured to display information such as power status, active phototherapy mode, selected wavelength, charging state, or fault conditions. For example, different colors or blinking patterns of the indicator lights may correspond to different operating modes or system states. This configuration enables users to easily understand device status at a glance, even during shower operation.

In an embodiment, the phototherapy unit may be arranged circumferentially around a water outlet side of the showerhead body. In this configuration, a plurality of light-emitting elements are positioned to surround the water outlet openings such that therapeutic light is emitted concurrently with water discharge. This arrangement allows the phototherapy unit to deliver uniform light exposure over a treatment region while maintaining normal shower functionality and further enables efficient integration of the phototherapy unit with the water outlet structure without obstructing water flow.

The present invention relates to a showerhead comprising a showerhead body and a phototherapy unit detachably mounted thereto. The showerhead body includes a housing defining a water cavity, a water outlet side, and a mounting hole configured to receive the phototherapy unit. The phototherapy unit is configured to emit therapeutic light toward a user during shower operation, thereby enabling simultaneous water delivery and phototherapy. The device may incorporate various structural, electrical, optical, and control features, including detachable locking structures, power supply units, control mechanisms, optical distribution elements, and safety features, which collectively enhance functionality, reliability, and ease of use.

The present invention has clear industrial applicability and may be manufactured using conventional sanitary ware and electronic production processes. The disclosed showerhead is suitable for use in residential bathrooms, hotels, healthcare facilities, wellness centers, spas, and other commercial or medical environments, providing enhanced personal care and therapeutic benefits without requiring additional space or separate devices. Owing to its modular design, scalability, and compatibility with existing shower installations, the invention is well-suited for mass production and commercial deployment. In an embodiment, the shower head system is designed in accordance with recognized electrical safety, photobiological safety, and usability engineering standards. The structural, electrical, and optical components may be configured to satisfy applicable safety requirements for consumer and therapeutic devices, thereby enhancing reliability, user safety, and compatibility with regulatory approval processes.

Various modifications to these embodiments are apparent to those skilled in the art from the description and the accompanying drawings. The principles associated with the various embodiments described herein may be applied to other embodiments. Therefore, the description is not intended to be limited to the embodiments shown along with the accompanying drawings but is to provide the broadest scope consistent with the principles and the novel and inventive features disclosed or suggested herein. Accordingly, the invention is anticipated to hold on to all other such alternatives, modifications, and variations that fall within the scope of the present invention and appended claims.

## Claims

1. A showerhead, comprising:
a showerhead body including a housing defining a water cavity and a water outlet side, the housing having a plurality of water outlet holes communicating with the water cavity on the water outlet side, and a mounting hole formed on the water outlet side, wherein the plurality of water outlet holes surrounds the mounting hole; and
a phototherapy unit detachably disposed in the mounting hole, wherein the phototherapy unit is configured to emit therapeutic light outwardly from the mounting hole toward a user.

2. The showerhead of claim 1, wherein the phototherapy unit is engaged with a wall of the mounting hole via a locking protrusion on the wall of the mounting hole and a locking groove on an outer peripheral side of the phototherapy unit.

3. The showerhead of claim 2, wherein:
in a depth direction of the mounting hole, the phototherapy unit includes a light-emitting side and a backlight side opposite to each other;
the locking groove includes a sliding groove section, a connecting groove section, and a locking groove section connected in sequence;
the sliding groove section and the locking groove section both extend along the depth direction of the mounting hole;
the connecting groove section extends circumferentially along the mounting hole;
one end of the sliding groove section is located on the backlight side, and another end of the sliding groove section is located between the light-emitting side and the backlight side; and
the connecting groove section and the locking groove section are located between the light-emitting side and the backlight side.

4. The showerhead of claim 2, wherein a plurality of locking protrusions are provided, a plurality of locking grooves is provided, one locking protrusion is correspondingly provided in one locking groove, and the plurality of locking protrusions are arranged sequentially along a circumference of the mounting hole.

5. The showerhead of claim 1, wherein in a depth direction of the mounting hole, the phototherapy unit includes a light-emitting side and a backlight side opposite to each other, and an outer diameter of the phototherapy unit gradually decreases in a direction from the light-emitting side to the backlight side.

6. The showerhead of claim 1, wherein the mounting hole is configured as a blind hole, and an exhaust gap is provided between the outer peripheral side of the phototherapy unit and a wall of the mounting hole, the exhaust gap being in communication with an environment outside the mounting hole.

7. The showerhead of claim 1, wherein the heat generated by the phototherapy unit is utilized to heat the water flowing through the water cavity.

8. The showerhead of claim 1, wherein the showerhead body further comprises a filter disposed along a water inlet flow path, the filter being positioned such that incoming water passes through the filter before reaching the water outlet side.

9. The showerhead of claim 1, wherein the phototherapy unit comprises a plurality of light-emitting diodes configured to emit light at two different wavelengths.

10. A showerhead system, comprising:
a housing defining a water cavity and a water outlet side, the housing having a plurality of water outlet holes on the water outlet side communicating with the water cavity, and a mounting hole formed on the water outlet side, wherein the plurality of water outlet holes surrounds the mounting hole;
a phototherapy unit detachably received within the mounting hole, the phototherapy unit being configured to emit therapeutic light outwardly from the mounting hole toward a user; and
a ball joint rotatably disposed on the housing and configured for connection to a water supply pipe.

11. The showerhead system of claim 10, wherein the phototherapy unit is engaged with a wall of the mounting hole via a locking mechanism comprising a locking protrusion on the wall of the mounting hole and a locking groove on the outer peripheral side of the phototherapy unit.

12. The showerhead system of claim 10, wherein the mounting hole is configured as a blind hole, and an exhaust gap is provided between the outer peripheral side of the phototherapy unit and a wall of the mounting hole, the exhaust gap being in communication with an environment outside the mounting hole.

13. The showerhead system of claim 10, wherein the phototherapy unit is configured to terminate operation upon completion of a predetermined treatment session and includes a feedback mechanism configured to notify the user that the treatment session has ended.

14. The showerhead system of claim 10, wherein the phototherapy unit is configured to operate in a plurality of selectable treatment durations, and upon selecting a desired treatment duration, the phototherapy unit automatically operates for the specified time period and subsequently shuts down.

15. The showerhead system of claim 10, further comprising a remote control configured to wirelessly communicate with the phototherapy unit, the remote control including a plurality of user-operable buttons to remotely power the phototherapy unit on or off and to control treatment parameters.

16. A showerhead, comprising:
a showerhead body including a housing having a mounting hole configured as a blind hole, the mounting hole having a wall provided with a locking protrusion; and
a phototherapy unit detachably disposed in the mounting hole, the phototherapy unit having an outer peripheral side provided with a locking groove, wherein the locking protrusion engages with the locking groove to secure the phototherapy unit within the mounting hole, wherein an exhaust gap is provided between the outer peripheral side of the phototherapy unit and the wall of the mounting hole, the exhaust gap being in communication with an environment outside the mounting hole and is used to dissipate the heat generated by the phototherapy unit, and wherein the phototherapy unit is configured to emit therapeutic light outwardly from the mounting hole toward a user.

17. The showerhead of claim 16, wherein:
in a depth direction of the mounting hole, the phototherapy unit includes a light-emitting side and a backlight side opposite to each other;
the locking groove includes a sliding groove section, a connecting groove section, and a locking groove section connected in sequence;
the sliding groove section and the locking groove section both extend along the depth direction of the mounting hole; and
the connecting groove section extends circumferentially along the mounting hole.

18. The showerhead of claim 16, wherein the housing defines a water cavity and a water outlet side, the housing has a plurality of water outlet holes communicating with the water cavity on the water outlet side, the mounting hole is formed on the water outlet side, and the plurality of water outlet holes surround the mounting hole.

19. The showerhead of claim 16, wherein the phototherapy unit comprises an audible notification device configured to emit an audible signal upon at least one of powering on the phototherapy unit, powering off the phototherapy unit, completion of a treatment session, or detection of a low-battery condition.

20. The showerhead of claim 16, wherein the phototherapy unit comprises at least two indicator lights configured to display battery status information, wherein the indicator lights operate in different illumination patterns to represent varying battery charge levels.
